# EUROPEAN PATENT APPLICATION

(11) **EP 1 166 774 A1**
(43) Date of publication of application: **02.01.2002**
(21) Application number: 01202415.4
(22) Date of filing: 22.06.2001
(51) Int. Cl.: A61K 9/12

(54) **Pharmaceutical aerosol compositions comprising a flavouring oil**

(30) Priority: 22.06.2000 GB 0015361
(71) Applicant: Pharmasol Limited, Andover, Hants SP10 5AZ (GB)
(72) Inventor: Ross, Calvin, Sulfolk IP27 0RB (GB)
(74) Representative: Hughes, Siân Victoria

(57) **Abstract**

The present invention relates to the use of a flavouring oil in a pharmaceutical aerosol formulation, preferably for sublingual delivery. The flavouring oil acts as a lubricant of the valve of the dispensing device used to administer said formulation. Preferably, the flavouring oil is a volatile oil and therefore also acts as a penetration enhancer.

## Description

The present invention relates to improvements in pharmaceutical compositions and, in particular, to an improved formulation for aerosol delivery of pharmaceutically active agents.

The formulations of the present invention are particularly well suited to sublingual aerosol delivery, although they can also be used in connection with other modes of aerosol delivery, such as inhalation.

It is known to spray compositions containing pharmaceutically active agents on to the sublingual mucosa. This mode of administration presents a number of advantages over other modes, such as oral administration, injection or inhalation.

Sublingual delivery of a pharmaceutically active agent results in fast uptake of the active agent and rapid onset of the therapeutic effect. The active agent is administered to the oral and sublingual mucosa, from which it is rapidly absorbed into the bloodstream. Sublingual delivery also avoids first-pass metabolism of the active agent, which is a recognised disadvantage encountered with oral administration. Sublingual delivery is painless and allows easy self-administration by the patient, unlike injection. Sublingual delivery also does not suffer from the disadvantages associated with inhalation or lung delivery, such as irritation of the trachea and lungs and involuntary coughing.

A sublingual dose is also absorbed at a predictable rate and so its administration can be accurately controlled. In particular, sublingual aerosol delivery is favoured.

It is known to use dispensing devices with metered valves in order to administer formulations containing pharmaceutically active agents sublingually, allowing accurate volumes, and therefore accurate doses, to be dispensed.

Generally, the formulations for sublingual administration comprise a pharmaceutically active agent and, optionally, a carrier. The device for aerosol delivery of a sublingual formulations may be a pump spray, or alternatively, the formulation may include a propellant.

Whilst the formulation is to contact the mucosa under the tongue, the patient will inevitably taste the formulation upon such administration, which, in many cases, will not be palatable. Not only does the taste of the formulation make the administration of the active agent unpleasant, it will also discourage the patient from administering the formulation, which could be dangerous in some cases where patient compliance is important.

It is known to include flavouring oils to mask the taste of the formulation to be administered sublingually. Peppermint oil is one of the preferred flavouring oils use in sublingual formulations, although clearly other flavoured oils may be used. However, whilst the flavouring oils have, up until now, been included in order to mask the taste of the formulation to be administered, the applicants have now found that their inclusion can have other beneficial effects which were not previously recognised.

It is essential that the metering valve of the dispensing device works properly in order to dispense the formulation in accurate and consistent doses over an extended period of time and after repeated administrations. The metering valve comprises moving parts and friction occurs between these parts which may lead to jamming and/or damage to the valve parts. It is therefore essential to lubricate the valve, to ensure that the valve works properly and consistently, and does not become damaged.

It is known to add lubricants to a pharmaceutical formulation in order to lubricate the valve parts. These known lubricating additives serve no other purpose and add to the cost of the formulation.

The applicants have found that a flavouring oil included in the formulation to be dispensed also lubricates the valve of the device used to dispense the formulation. This lubrication thereby helps the smooth and reliable operation of the valve and the dispensing device as a whole. Thus, the inclusion of a flavouring oil not only masks the unpleasant taste of the formulation to be administered under the tongue, but also lubricates the valve, thereby extending the life of the dispensing device and enhancing its accuracy, whilst avoiding the need to include a separate lubricating additive.

The preferred oil for this dual purpose is peppermint oil, because it is palatable to the majority of people, it masks other tastes well, and it acts well as a valve lubricant.

Volatile oils in formulations for sublingual delivery act as penetration enhancers. Thus, they increase the absorption of the active agent at the sublingual mucosa and making the administration more effective because more of the active agent contained in the administered formulation passes through the mucosa and into the bloodstream.

Thus, if a flavouring oil which is also a volatile oil is added to the formulation, this will have three beneficial properties or effects. It will mask the taste of the formulation, it will lubricate the valve of the dispensing device and it will act as a penetration enhancer. This will overcome the need for separate additives for each of these three purposes, reducing the cost of the formulation.

Peppermint oil is a volatile oil, as is eucalyptus oil, both of which may therefore be used to achieve the three beneficial effects discussed above. It is, of course, also possible to combine two or more different oils (such as a non-volatile flavouring oil and a volatile oil) to achieve the same three effects.

In a preferred embodiment of the present invention, the formulation is administered using an aerosol device. The spray device may be a pump spray or, alternatively, the formulation may include a propellant.

The oils described above are added directly to the formulations and do not react with the other formulation constituents, which may include a propellant such as, for example, 1,1,1,2-tetrafluoroethane (HFC-134a), 1,1,1,2,3,3,3-heptafluoropropane (HFC-227) or a combination thereof, or butane.

Whilst, in some circumstances, the formulation may comprise just the active agent, an oil such as peppermint oil and a propellant if appropriate, the formulation will preferably also include a carrier. In a preferred embodiment of the invention, the carrier is a lower alkyl (C₁-C₄) alcohol, a polyol, or a (poly) alkoxy derivative. In embodiments, the carrier is a C₁-C₄ alkyl alcohol or a lanolin alcohol and, preferably, is ethanol or isopropyl alcohol. The most preferred alcohol is ethanol.

The preferred polyols include propylene glycol and glycerol and the preferred (poly) alkoxy derivatives include polyalkoxy alcohols, in particular 2-(2-ethoxyethoxy) ethanol (available under the Trademark Transcutol ® ).

Further preferred (poly)alkoxy derivatives include polyoxyalkyl ethers and esters, such as polyoxyethylene ethers or esters. The preferred polyoxyethylene ethers and esters are polyoxyethylene alkyl ethers, polyoxyethylene castor oil derivatives, polyoxyethylene sorbitan fatty acid esters and polyoxyethylene stearates.

The preferred fatty acid alkyl esters are ethyl oleate, isopropyl myristate and isopropyl palmitate. The preferred polyalkylene glycol is polyethylene glycol.

In further embodiments, compositions used in the present invention can comprise a plurality of different carriers. Other substances or additives may also be included, according to the requirements of the particular active agent, etc..

In one embodiment of the invention, the pharmaceutically active agent included in the formulation is not an opioid analgesic.

Devices suitable for providing sublingual aerosol delivery of formulations of the present invention include those disclosed in WO 92/11190, US-A-4819834 and US-A-4407481. Many of these devices include metering valves having components formed from plastic materials, such as the valves available from Bespak PLC of Bergen Way, Kings Lynn, Norfolk PE30 2JJ, United Kingdom, in which the valve core, metering chamber and some other structural components are formed from plastic materials. The plastic materials currently used for forming these structural parts in valves employed with many chlorofluorocarbon containing formulations include certain acetal co-polymers.

Although the plastics employed to manufacture metering valves, including the aforementioned acetal co-polymers, have also been found to be stable in the presence of HFC-134a alone, the applicants, to their surprise, have determined that many of these plastics materials can be caused to swell in the presence of formulations which include certain constituents with HFC-134a. When such swelling takes place in a valve, the fit of mutually slidable components, such as metering chambers and valve cores, is adversely effected and they can bind together or become loose, causing the valve to leak or cease functioning altogether.

This problem has now been solved by using a device for providing pharmaceutical doses comprising a container, filled with a pharmaceutical composition including a pharmaceutically active agent in a solution of liquefied HFC-134a, or HFC-227, and a carrier selected from pharmaceutically acceptable alcohols, polyols, (poly)alkoxy derivatives, fatty acid alkyl esters, polyalkylene glycols, and dimethylsulphoxide, and valve means arranged for delivering aerosol doses of said pharmaceutical composition to the exterior of the container, wherein at least a portion of the device is formed from a polyester. Preferably, the valve means includes at least one component formed from a polyester, which component, more preferably, is a metering chamber and/or a valve core.

In further embodiments, the container comprises a polyester and, preferably, consists of metal lined with a polyester. The canister cap can also be so formed.

Apart from allowing the aforementioned swelling problem to be solved, an advantage of this aspect of the present invention is that use of expensive metal valve components can be avoided.

The preferred polyesters are polyalkylene benzene dicarboxylates, more preferably polyalkylene terephthalates and, most preferably, a polybutylene terephthalate.

Such materials, preferably, have a density of about 1.3g/cm³ and a water absorption of about 0.6% (23°C saturation). The polyesters, also, are preferably partially crystalline in nature and have a crystalline melting range of 220-225°C.

Examples of suitable polybutylene terephthalates include those available under the Trademark Celanex® from Hoechst UK Limited, Walton Manner, Milton Keynes, Bucks MK7 7AJ, United Kingdom. Particularly preferred are Celanex® 2500 and Celanex® X 500/2.

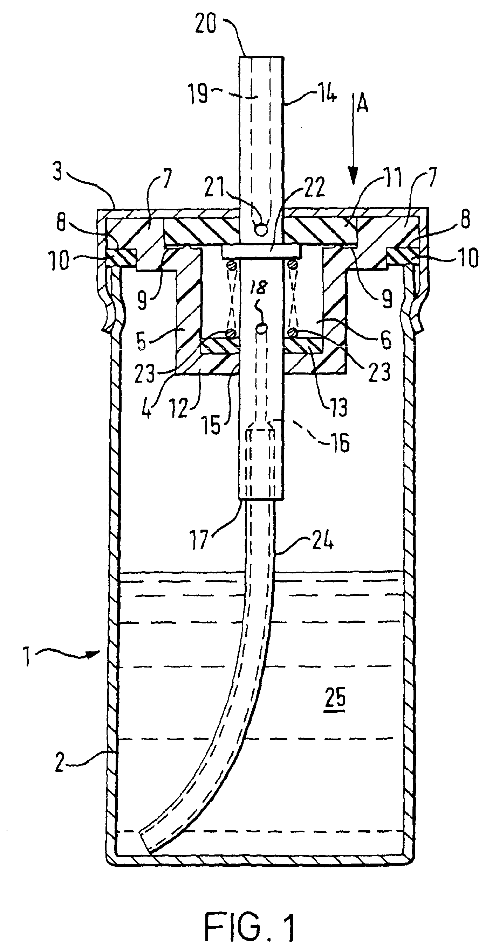

Figure 1 is a cross sectional view of an embodiment of a device in accordance with the invention.

The device 1 comprises a substantially cylindrical canister 2 sealed with a cap 3. Both the canister 2 and the cap 3 may be manufactured from a variety of materials. The canister and cap may be lined with a polyester (such as Celanex® 2500) or a lacquer (not shown).

A valve body moulding 4 comprises a cylindrical portion 5, which defines a metering chamber 6 and a stepped flange portion 7, and is formed by injection moulding from Celanex® 2500. The stepped flange portion 7 defines a first and outwardly facing annular seat 8 and a second, inwardly facing annular seat 9. The first annular seat 8 accommodates an annular sealing ring 10 and the second annular seat 9 accommodates a first sealing washer 11. The first sealing washer 11 is located so as to cooperate with the cylindrical portion 5 of the valve body moulding 4, in defining the metering chamber 6.

A base 12 of the cylindrical portion 5 of the valve body moulding 4 completes the boundary to the metering chamber 6 and provides a seat for a second sealing washer 13.

The sealing ring 10 and the first and second sealing washers 11 and 13 can be formed from a butyl rubber, neoprene or one of the elastomers disclosed for such purposes in WO 92/11190.

An elongate, substantially cylindrical and partially hollow valve core 14 is slidably located within the first and second sealing washers 11 and 13 and extends through an orifice 15, defined in the base 12. The valve core 14 is formed by injection moulding from Celanex® 2500.

A stepped inlet passage 16 communicates between a first end 17 of the valve core 14 and an inlet orifice 18, formed through the side of the valve core 14. In a likewise manner, an outlet passage 19 communicates between the second end 20 of the valve core 14 and an outlet orifice 21 formed through the side of the valve core 14. An annular flange 22 extends radially outwardly from the valve core 14 between the inlet and outlet orifices 18 and 21 and adjacent to the outlet orifice 21.

A stainless steel compression coil spring 23 acts between the annular flange 22 and the second sealing washer 13, urging the annular flange 22 into contact with the first sealing washer 11, such that the outlet orifice 21 lies inside the first sealing washer 11 and is thereby isolated from the metering chamber 6. In this position, as shown in Figure 1, the inlet orifice 18 is located within the metering chamber 6. A flexible tube 24 is engaged within the stepped inlet passage 16 and extends from the valve core 14 to the base of the canister 2 (as shown in Figure 1). Thus, the inlet orifice 18 is in communication with a region within the canister 2 adjacent to its base 12.

The cap 3 is firmly attached to the canister 2 by crimping and, thus, holds the assembly of the valve body moulding 4, valve core 14, coil spring 23, sealing washers 11 and 13 and sealing ring 10 in place as shown in Figure 1, with the sealing ring 10 and first sealing washer 11 sufficiently compressed to seal the interior of the device 1 and prevent the egress of its contents.

Downward movement of the valve core, in the direction of arrow A, against the action of the spring 22 will bring the outlet orifice 21 into the metering chamber immediately after the first orifice 18 has been sealed from the metering chamber 6 by the second sealing washer 13.

When filled with a composition in accordance with the present invention, as shown at 25, the device 1 will provide metered doses of the composition when used as follows. The device 1 should be held in the position shown in Figure 1, so that the composition 25, by virtue of its pressure, enters the metering chamber 6 via the tube 24, the inlet passage 16 and the inlet orifice 18. Subsequent depression of the valve core 14, in the direction of arrow A, seals the inlet orifice 18 and hence the remainder of the canister 2, from the metering chamber 6 and opens the outlet passage to the metering chamber 6, via the outlet orifice 21. Since the composition 25 in the metering chamber 6 is pressurised with the propellant, it will be expelled from the metering chamber 6 through the outlet orifice 21 and the outlet passage 19. If the valve core 14 is then allowed to return to the position shown in Figure 1, under the influence of the spring 22, the outlet orifice 21 is again sealed from the metering chamber 6 and the metering chamber 6 will be filled with pressurised composition 25 from the canister 2, via the tube 24, stepped inlet passage 16 and inlet orifice 18.

There now follows an example of a composition according to the present invention for sublingual delivery.

### Example

A composition comprising a pharmaceutically active agent with HFC-134a suitable for use in a device as described above can be formulated from the following ingredients:-

| Component | percent w/w | g/can |
|---|---|---|
| Pharmaceutically active agent | 0.7 | 0.099 |
| Ethanol 96% BP | 13.2 | 1.866 |
| Peppermint oil | 1.4 | 0.205 |
| HFC-134a | 84.7 | 12.02 |
| Total | 100 | 14.19 |

The peppermint oil is added to the active agent/ethanol solution and mixed thoroughly. 2.17g of the resulting solution is then placed in the canister 2 and the valve assembly, comprising the valve body moulding 4, first sealing washer 11, second sealing washer 13, spring 22, tube 23, and annular seal 10 are then sealed onto the canister 2 as shown in Figure 1 by the cap 3. The propellant is then added to the canister by being forced through the valve core 14 at great pressure, and the complete device is then checked for leaks.

Although only peppermint oil is referred to in the above-mentioned example, other flavouring oils may be substituted therefor. As discussed above, the flavouring oil is preferably also a volatile oil, so that it has the penetration enhancing effect upon sublingual administration.

## Claims

1. Use of a flavouring oil in a pharmaceutical aerosol formulation, for lubrication of the valve of a spray device used to administer said formulation.

2. A use as claimed in claim 1, wherein the formulation is for sublingual delivery.

3. A use as claimed in claim 1 or 2, wherein the flavouring oil is a volatile oil and acts as a penetration enhancer.

4. A use as claimed in any of the preceding claims, wherein the flavouring oil is peppermint oil.

5. A use as claimed in any of the preceding claims, wherein formulation comprises a pharmaceutically active agent and the flavouring oil.

6. A use as claimed in claim 5, wherein the formulation also includes a propellant.

7. A use as claimed in claim 6, wherein the propellant is 1,1,1,2-tetrafluoroethane (HFC-134a) or 1,1,1,2,3,3,3-heptafluoropropane (HFC-227), or a combination thereof.

8. A use as claimed in any of the preceding claims, wherein the formulation includes 5% by weight flavouring oil.

9. A use as claimed in any of the preceding claims, wherein the formulation further comprises a carrier selected from pharmaceutically acceptable alcohols, polyols, (poly) alkoxy derivatives, fatty acid alkyl esters, polyalkylene glycols, and dimethyl sulphoxide.

10. A use as claimed in claim 9, wherein the carrier is ethanol.

11. A use as claimed in claim 9 or 10, comprising up to 50% w/w, preferably up to 25% w/w carrier.

12. A use as claimed in any one of claims 9-11, comprising a plurality of carriers.

13. A use as claimed in any of the preceding claims, wherein the formulation further comprises a surfactant.

14. A use as claimed in any of the preceding claims, in the absence of another lubricant.

15. A use as claimed in any of the preceding claims, in the absence of another penetration enhancer.

16. A use as claimed in any of the preceding claims, wherein the dispensing device comprises a container filled with the formulation and the valve means is arranged for delivering aerosol doses of said formulation to the exterior of the container.

17. A use as claimed in claim 16, wherein at least a portion of the device is formed from a polyester.

18. A use as claimed in claim 16 or 17, wherein the valve means includes at least one component formed from a polyester.

19. A use as claimed in claim 18, wherein two relatively movable, preferably. mutually slidable, valve components are formed from a polyester.

20. A use as claimed in claim 18 or 19, wherein the valve core is formed from polyester.

21. A use as claimed in any one of claims 18-20, wherein the metering chamber of the valve is formed of polyester.

22. A use as claimed in any one of claims 17-21, wherein the polyester is a polyalkylene benzene dicarboxylate.

23. A use as claimed in claim 22, wherein the polyester is a polyalkylene terephthalate, and more preferably, is a polybutylene terephthalate.
